# EUROPEAN PATENT SPECIFICATION

(11) **EP 4 554 470 B1**
(45) Date of publication and mention of the grant of the patent: **22.04.2026**
(21) Application number: 23741051.9
(22) Date of filing: 12.07.2023
(51) Int. Cl.: A61B 5/11, A61B 5/02, A61B 5/00

(54) **AUTOENCODERS IN QUANTITATIVE SEISMOCARDIOGRAPHY**
AUTOCODIERER FÜR DIE QUANTITATIVE SEISMOKARDIOGRAFIE
AUTO-CODEURS EN SÉISMOCARDIOGRAPHIE QUANTITATIVE

(30) Priority: 13.07.2022 EP 22184623
(43) Date of publication of application: 21.05.2025
(73) Proprietor: Acarix A/S, 2900 Hellerup (DK)
(72) Inventor: SCHMIDT, Samuel Emil, 9210 Aalborg SØ (DK)
(74) Representative: Öberg & Sjöberg AB
(86) International application number: PCT/EP2023/069321
(87) International publication number: WO 2024/013233

(56) References cited:
- WO-A1-2020/245340
- OMER T. INAN ET AL: "Novel Wearable Seismocardiography and Machine Learning Algorithms Can Assess Clinical Status of Heart Failure Patients", CIRCULATION. HEART FAILURE (PRINT), vol. 11, no. 1, 12 January 2018 (2018-01-12), US, XP055629467, ISSN: 1941-3289, DOI: 10.1161/CIRCHEARTFAILURE.117.004313
- ZIA JONATHAN ET AL: "Modeling Consistent Dynamics of Cardiogenic Vibrations in Low-Dimensional Subspace", IEEE JOURNAL OF BIOMEDICAL AND HEALTH INFORMATICS, IEEE, PISCATAWAY, NJ, USA, vol. 24, no. 7, 16 March 2020 (2020-03-16), pages 1887 - 1898, XP011796254, ISSN: 2168-2194, [retrieved on 20200701], DOI: 10.1109/JBHI.2020.2980979
- TILENDRA CHOUDHARY ET AL: "Design of Breathing-states Detector for m-Health Platform using Seismocardiographic Signal", ARXIV.ORG, CORNELL UNIVERSITY LIBRARY, 201 OLIN LIBRARY CORNELL UNIVERSITY ITHACA, NY 14853, 5 April 2021 (2021-04-05), XP081923189, DOI: 10.1109/JSEN.2020.3025384
- HAESCHER MARIAN ET AL: "Transforming Seismocardiograms Into Electrocardiograms by Applying Convolutional Autoencoders", ICASSP 2020 - 2020 IEEE INTERNATIONAL CONFERENCE ON ACOUSTICS, SPEECH AND SIGNAL PROCESSING (ICASSP), IEEE, 4 May 2020 (2020-05-04), pages 4122 - 4126, XP033792787, DOI: 10.1109/ICASSP40776.2020.9053130

## Description

### Technical field of the Invention

The proposed technology relates to seismocardiography and techniques for assisting in diagnosing heart failure.

### Background of the Invention

Seismocardiography (SCG) is the analysis of sub-audible low-frequency vibrations at the chest wall caused by the beating heart. More generally, SCG relates to non-invasive measurement of accelerations in the chest wall produced by myocardial movement. Heart sounds are audible components of the chest wall vibrations that typically are above 40-60 Hz, while SCG frequencies are typically below 5 Hz.

SCG is typically measured using an accelerometer. However, when an accelerometer is used, both low frequency SCG components and audible components are simultaneously sampled. The SCG components and the audible components reveal different cardiovascular functions, thus enabling different approaches to diagnosing a cardiovascular function. For example, SCG is typically suitable for estimation of time intervals between features in the cardiac cycle, while heart sounds are appropriate for detection of murmurs caused by flow disturbances.

Heart failure does not have a single origin but can be caused by different underlying conditions. Therefore, heart failure can alter SCG signals in many ways. For example, amplitude measures can both increase and decrease due to heart failure. This poses a challenge in identifying heart failure in an SCG signal of a subject.

OMER T. INAN et al: "Novel Wearable Seismocardiography and Machine Learning Algorithms Can Assess Clinical Status of Heart Failure Patients", CIRCULATION. HEART FAILURE (PRINT), vol. 11, no. 1, 12 January 2018 (2018-01-12), US, ISSN: 1941-3289, DOI: 10.1161/CIRCHEARTFAILURE.117.004313; discloses remote monitoring of patients with heart failure using a wearable ECG and seismocardiogram sensing patch. To assess patient state, the similarity of the structure of seismocardiogram signals after exercise is compared with rest using graph mining (graph similarity score).

WO 2020/245340 A1 discloses technology related to quantifying of cardiorespiratory fitness by obtaining a seismocardiogram recorded with an accelerometer placed on a person. Properties of a first signal feature (AC) in the seismocardiogram (SCG) are determined, wherein the first signal feature (AC) corresponds to the aortic valve closure (AC) of a heartbeat. A measure indicating cardiorespiratory fitness (VO2max) is then determined based on the properties of first signal feature (AC). One of the properties is the morphology of the first signal feature represented by a morphology measure (ACMorphology). Determining the morphology measure involves a dimensionality reduction which may be performed using an auto encoder.

### Object of the Invention

An object of the present invention is to provide an improved technology for identifying heart failure. It is a further object to provide a technology that is reliable and easy to use.

### Summary of the Invention

The invention is defined as set out in the appended claims.

### Summary of Disclosed Technology

According to a first aspect of the proposed technology, a method for determining an indication of heart failure of a subject, or person, is proposed which is performed by the processor of the system of the present invention.

The method comprises:
*obtaining* a first signal interval from a source signal recorded with an accelerometer placed on the chest of a subject; *inputting* the first signal interval into a first autoencoder, wherein the first autoencoder is trained on the corresponding first signal intervals obtained from healthy subjects and outputs a reconstructed first signal interval; *determining* a first correlation, or first error, between the first signal interval and the reconstructed first signal interval; and
*determining* the indication of heart failure based on the first correlation. Worded differently, a method is proposed for determining an indication of heart failure of a subject, or person, based on a first signal interval from a source signal recorded with an accelerometer placed on the chest of a subject, wherein the method comprises: *inputting* the first signal interval into a first autoencoder, wherein the first autoencoder is trained on the corresponding first signal intervals obtained from healthy subjects and outputs a reconstructed first signal interval; *determining* a first correlation, or first error, between the first signal interval and the reconstructed first signal interval; and *determining* the indication of heart failure based on the first correlation.

Wording the last two steps of the methods differently, the method comprises: *determining, or estimating,* the indication of heart failure based on a correlation, or error, between the first signal interval and the reconstructed first signal interval. The first signal interval may correspond, or correlate in time, to a first subinterval of a heart cycle. Alternatively, it may correspond to, or correlate in time, to a complete heart cycle.

According to a second aspect of the proposed technology, a system for determining an indication of heart failure of a subject is proposed, wherein the system comprises: (A) an accelerometer configured to be placed on the chest of a subject, and (B) a processor operatively connected to the accelerometer and configured to perform the method according to the first aspect of the proposed technology.

According to a third aspect of the proposed technology, a system for determining an indication of heart failure of a subject is proposed. The system comprises a plurality of modules. It is understood that the modules jointly perform the, or is configured to jointly perform, the method according to the first aspect of the proposed technology, wherein each module performs one of the steps specified in relation to the first aspect of the proposed technology.

According to a fourth aspect of the proposed technology, a computer program product is proposed for being used in a system for determining an indication of heart failure of a subject. The system comprises: (A) an accelerometer for being placed on the chest of a subject; and (B) a processor operatively connected to the accelerometer. The computer program product comprising program code instructions configured to, when executed by the processor of the system, cause the processor to perform the method according to the first aspect of the proposed technology.

According to a fifth aspect of the proposed technology, a non-transient memory is proposed on which a computer program product according to the fourth aspect of the proposed technology is stored.

The first autoencoder is trained on healthy subjects. This means that it is efficient in reconstructing first signal intervals from healthy subjects and that it is less efficient in reconstructing the often more complex first signal intervals of subjects with heart failure. The first correlation is then lower for subjects with heart failure. This difference in correlation is utilized when determining the indication of heart failure.

It is understood that the accelerometer is configured for measuring accelerations and vibrations of the chest wall of the subject that are caused by myocardial movement. That the first signal interval corresponds to a first subinterval of a heart cycle means that it covers, or corresponds to, a first portion of a single heart cycle.

In the method of the first aspect, the accelerometer may be placed on the chest of a subject and attached to the skin of the subject by an adhesive for measuring the accelerations and vibrations. The systems of the second, third and fourth aspects may further comprise an adhesive patch configured for supporting the accelerometer, or the housing described below, and for being attached to the skin of the subject. By attaching the accelerometer, or housing, to the skin, the quality of the recorded signals is improved.

The accelerometer may be placed on the front of the chest of the subject. The accelerometer being placed on the chest of a subject means that it is placed on the outside and not on the inside of the body. This has the advantage of a simple application that does not require trained staff. It also has the advantage that no invasive procedure is required and that it can be used in non-sterile environments.

The accelerometer may comprise a piezoelectric element. The signal may represent a voltage generated by the piezoelectric element. Thus, the signal strength or amplitude of the source signal may represent a voltage value.

The different aspects described above may be modified as described below.

The first signal interval, or first subinterval, may cover the systole, or a portion of the systole, of the heart cycle. Alternatively, it may cover the diastole, or a portion of the diastole, of the heart cycle.

The first autoencoder may compress the first signal interval to a number of nodes, or variables, wherein the number of nodes is less than 15, less than 12, or less than 10, or in the range 5 to 15, 6 to 12, or 7 to 9. The first autoencoder may be a single layer autoencoder. Preferably, the first autoencoder is an Undercomplete Autoencoder, meaning that the number of layers of the first autoencoder is smaller than the number of samples in the signal interval. Alternatively, the first autoencoder is a Sparse Autoencoder, Convolutional Autoencoder, Variational Autoencoder, Contractive Autoencoder, or Deep Autoencoder.

The first correlation may be a correlation measure, or error measure, between the first signal interval and the reconstructed first signal interval, for example based on a Pearson correlation coefficient, a Mean-Square Error (MSE), or a Root-Mean-Square Error (RMSE). Worded differently, determining the first correlation may comprise: *determining* a correlation measure, or error measure, between the first signal interval and the reconstructed first signal interval. For example, the correlation measure may be a Pearson correlation coefficient, a spearman correlation coefficient, a MSE, or a RMSE between the first signal interval and the reconstructed first signal interval.

The indication of heart failure may be a measure, such as a measure that indicates the likelihood for the subject having heart failure. The indication of heart failure may be a probability score, or risk score, for heart failure, for example based on a logistic regression model and the first correlation. Worded differently, determining the indication of heart failure may comprise: *determining* a probability score, or risk score, for heart failure, for example based on a logistic regression model and the first correlation. *Determining* a probability score, or the probability score, may further be based on demographic data, such as gender and age.

It is specified above that the first signal may correspond to a first subinterval of a heart cycle. The method may further comprise: *obtaining* a second signal interval from the source signal recorded with an accelerometer placed on the chest of a subject, wherein the second signal interval corresponds, or correlates in time, to a second subinterval of a heart cycle; *inputting* the second signal interval into a second autoencoder, wherein the second autoencoder is trained on the corresponding second signal intervals obtained from healthy subjects and outputs a reconstructed second signal interval; and *determining* a second correlation, or second error, between the second signal interval and the reconstructed second signal interval. In the alternative wording of the first aspect of the proposed technology, the method may further be based on a second signal interval from the source signal, wherein the second signal interval corresponds, or correlates in time, to a second subinterval of a heart cycle, wherein the method further comprises: *inputting* the second signal interval into a second autoencoder, wherein the second autoencoder is trained on the corresponding second signal intervals obtained from healthy subjects and outputs a reconstructed second signal interval; and *determining* a second correlation, or second error, between the second signal interval and the reconstructed second signal interval.

Determining the indication of heart failure may then be further based on the second correlation. Wording the determining of the second correlation and the indication differently, the method may comprise: *determining* the indication of heart failure based on the correlation, or error, between the first signal interval and the reconstructed first signal interval and between the second signal interval and the reconstructed second signal interval. Alternatively, the indication based on the first signal interval may be a first indication and the indication based on the second signal interval may be second indication that is independent from the first indication.

The first and second subintervals of the heart cycle may overlap. The first signal interval, or first sub interval, may have a center within the systole of the heart cycle, or cover the systole, and the second signal interval, or second subinterval, may have a center within the diastole of the heart cycle, or cover the diastole. It has been found that this contributes to an improved indication of heart failure.

The second autoencoder may compress the first signal interval to a number of nodes, or variables, wherein the number of nodes is less than 15, less than 12, or less than 10, or in the range 5 to 15, 6 to 12, or 7 to **9.** The second autoencoder may be a single layer autoencoder. The second autoencoder may be any of the types of autoencoders mentioned above in relation to the first autoencoder. For example, it may be an Undercomplete Autoencoder. The first autoencoder and the second autoencoder may be of the same type.

The second correlation may be a correlation measure, or error measure, between the second signal interval and the reconstructed second signal interval, for example based on a Pearson correlation coefficient, MSE, or RMSE. Worded differently, determining the second correlation may comprise: determining a correlation measure, or error measure, between the second signal interval and the reconstructed second signal interval. For example, the correlation measure may be a Pearson correlation coefficient, spearman correlation coefficient, a MSE, or a RMSE between the second signal interval and the reconstructed second signal interval.

The indication of heart failure may be a probability score, or risk score, for heart failure, for example based on a logistic regression model, the first correlation, and the second correlation. Worded differently, determining the indication of heart failure may comprise: *determining* a probability score, or risk score, for heart failure based on a logistic regression model, the first correlation, and the second correlation.

It is understood that the source signal may extend, or be recorded over, a period covering a plurality of cardiac cycles. It is further understood that that the first signal interval may be determined from, or within, a mean segment based on, or from, a plurality of signal segments of the source signal, wherein each signal element covers a single cardiac cycle.

Obtaining the first signal interval may comprise: *recording* a source signal with an accelerometer placed on the chest of a subject, wherein the source signal is recorded over a period covering a plurality of cardiac cycles of the subject; *dividing* the source signal into a plurality of signal segments, wherein each signal segment covers a single cardiac cycle; *aligning* the plurality of signal segments; *determining* a mean segment based on, or from, the plurality of signal segments, and *determining* the first signal interval in the mean segment.

In the alternative wording of the first aspect of the proposed technology, the method may further comprise: *downloading* the source signal from a digital storage, such as a computer server system or cloud storage. The method may further comprise: *dividing* the source signal into a plurality of signal segments, wherein each signal segment covers a single cardiac cycle; *aligning* the plurality of signal segments; *determining* a mean segment based on, or from, the plurality of signal segments, and *determining* the first signal interval in the mean segment. It is understood that the digital storage may be at a remote location relative to the accelerometer that recorded the source signal. The source signal may have been obtained for a different purpose than determining a risk for heart failure. For example, it may have been recorded for investigations relating to seismocardiography (SCG) or ballistocardiography (BCG). This means that the proposed technology can be used in large statistical studies on stored data.

Dividing the source signal into a plurality of signal segments may comprise: *identifying* a plurality of heart sounds in the source signal. For example, the heart sounds may be the S1 or the S2 heart sounds. Each heart sound relates to a single cardiac cycle. The recorded source signal is divided into the plurality of segments based on the identified plurality of heart sounds. This may involve extracting an audio signal from the source signal and determining the heart sounds in the audible signal. For example, the audio signal may be extracted by filtering the source signal with a high-pass filter having a lower cut-off frequency in the range 40-60 Hz, or approximately equal to 50 Hz.

Alternatively, the method may further comprise: *recording* an audio signal with a microphone placed on the chest of the subject simultaneously to recording the source signal with the accelerometer, and dividing the source signal into a plurality of signal segments may comprise: identifying a plurality of heart sounds in the audio signal, wherein each heart sound relates to a single cardiac cycle, and dividing the source signal into the plurality of segments based on the identified plurality of heart sounds.

The first signal interval may cover the Aortic valve Opening (AO) of the heart cycle. Additionally, or alternatively, it may cover the Aortic valve Closure (AC) of the heart cycle.

Determining the first signal interval in the mean segment may comprise: *identifying* a first fiducial point in the mean segment; *positioning* the first signal interval relative to the first fiducial point. For example, the first fiducial point may be the Gs point in the systole, which for example is defined in Sørensen, K., Schmidt, S.E., Jensen, A.S. et al. Definition of Fiducial Points in the Normal Seismocardiogram. Sci Rep 8, 15455 (2018) (https://doi.org/10.1038/s41598-018-33675-6). The first fiducial point may be located at the Aortic valve Opening (AO) of the heart cycle. The first signal interval may have a fixed length. For example, it may have a length of 750 ms. The first signal interval may extend from - 250 to +500 ms relative to the Gs point.

Similar to the first signal interval, the second signal interval may be determined from, or within, the mean segment. Obtaining the second signal interval may comprise: *determining* the second signal interval in the mean segment. In the alternative wording of the first aspect of the proposed technology with the abovementioned downloading of the source signal, the method may comprise: *determining* the second signal interval in the mean segment. The second signal interval may cover the Aortic valve Closure (AC) of the heart cycle. This way, the second signal interval includes information regarding the effeminacy of diastolic relaxation, which is considered an imported parameter.

Determining the second signal interval in the mean segment may comprise: *identifying* a second fiducial point in the mean segment; and *positioning* the second signal interval relative to the second fiducial point. For example, the second fiducial point may be the Dd point in the diastole, which for example is defined in Sørensen, K., Schmidt, S.E., Jensen, A.S. et al. Definition of Fiducial Points in the Normal Seismocardiogram. Sci Rep 8, 15455 (2018) (https://doi.org/10.1038/s41598-018-33675-6). The second fiducial point may be located at the Aortic valve Closure (AC) of the heart cycle. The first signal interval may have a fixed length. For example, it may have a length of 750 ms. The first signal interval may extend from - 300 to +500 ms relative to the Ds point.

The method may further comprise: *outputting,* or displaying, the indication of heart failure. For example, the indication of heart failure may be displayed as a number, such as in the interval 0 to 100. The system may comprise a display for displaying the indication of heart failure.

The method may further comprise: *filtering* the source signal, the plurality of segments, or the mean segment with a high-pass filter having a cutoff frequency below 1. Worded differently, the source signal may be an SCG signal. The source signal may encompass sub-audible and audible frequency components.

The method may further comprise: *discarding* noisy signal segments. This may be done as specified in WO2017/216374 A1.

The system of the above aspects may comprise: (C) a non-transient memory storing program code instructions that, when executed by the processor, configures the processor to perform the method.

The system may comprise a smartphone. The processor and/or the non-transient memory may be integral parts of the smartphone. Further, the accelerometer may be an integral part of the smartphone. The system may also comprise a casing or holder for supporting the smartphone, and the casing or holder may comprise an adhesive patch configured for attaching the casing or holder to the skin of the subject. Alternatively to the accelerometer being an integral part of the smartphone, the accelerometer may form part of an auxiliary unit configured to communicate with the smartphone by wire or wirelessly, such as a band that can be strapped around the chest of subject.

The processor may be further configured to: operate the accelerometer for recording a source signal, for example with the accelerometer placed on the chest of a subject, wherein the signal is recorded over a period of time covering a plurality of cardiac cycles of the subject. The processor may be further configured to: divide the recorded signal into a plurality of signal segments.

The system of the above aspects may comprise: (D) a microphone configured to be placed on the chest of the subject for measuring sounds generated by the beating heart, wherein the processor is further operatively connected to the microphone and configured to: operate the accelerometer to record a source signal with the accelerometer, **e.g.** placed on the chest of a subject; operate the microphone to record an audio signal with the microphone placed on the chest of a subject simultaneously to the source signal being recorded with the accelerometer; identify a plurality of heart sounds in the audio signal, wherein each heart sound relates to a single cardiac cycle; and divide the recorded source signal into the plurality of segments based on the identified plurality of heart sounds to obtain the plurality of signal segments. Alternatively, the processor may be further configured to: operate the accelerometer to record a source signal, **e.g.** with the accelerometer placed on the chest of a subject; filtering the source signal to obtain an audio signal; identify a plurality of heart sounds in the audio signal, wherein each heart sound relates to a single cardiac cycle; and divide the recorded signal into the plurality of signal segments based on the identified plurality of heart sounds. The filtering may comprise a high-pass filter having lower cut-off frequency in the range 40-60 Hz, or approximately equal to 50 Hz.

Here, the plurality of heart sounds may be the first heart sound (S1). Alternatively, the plurality of heart sounds may be the second heart sound (S2).

The system may comprise a housing or cover that supports and encloses or covers the processor. The housing or cover may further enclose or cover at least a portion of, or the whole of, the accelerometer and/or enclose or cover at least a portion of, or the whole of, the microphone.

Obtaining the first signal interval, and optionally the second signal interval, may further comprise: *storing* the plurality of signal segments, and/or the mean in the non-transient memory or in an auxiliary non-transient memory. The auxiliary non-transient memory may form part of computer server system, which may be at a remote location.

Determining the indication of heart failure may comprise: *storing* the indication of heart failure, or storing the first indication and the second indication of heart failure, in the non-transient memory or in an auxiliary non-transient memory. The auxiliary non-transient memory may form part of computer server system, which may be at a remote location.

Outputting, or displaying, the indication of heart failure may further comprise: *obtaining* a previously determined indication of heart failure for the subject and the indication of heart failure output information may further be based on the previously obtained indication of heart failure. The previously obtained measure may be stored in the non-transient memory or in the auxiliary non-transient memory. Outputting, or displaying, the indication may further comprise: *outputting,* or displaying, the previously determined indication of heart failure.

The previously determined indication of heart failure may have been determined in the same manner as the indication of heart failure. The previously determined indication of heart failure may have been determined at an earlier point in time, such as more than five days or ten days prior to determining the measure or the mean segment.

Further advantages with and features of the different aspects will be apparent from the following description of the drawing.

### Brief description of the drawings

A more complete understanding of the abovementioned and other features and advantages of the present invention will be apparent from the following detailed description of the drawings, wherein:
Fig.1 is a schematic illustration of an embodiment of a system for determining an indication of heart failure,
Fig.2 is a flow chart illustrating the steps of a method performed by the system described in relation to Fig.1,
Fig.3 shows a portion of an audio signal,
Fig.4 shows a mean segment with first and second signal intervals indicated by dashed lines,
Fig.5 is a schematic illustration of an autoencoder,
Fig.6 is a schematic illustration of an alternative embodiment of a system for determining an indication of heart failure,
Figs.7a and 7b are schematic illustrations of alternative embodiment of a system for determining an indication of heart failure,
Figs.8a and 8b are graphs showing the first signal interval (solid) and the reconstructed first signal interval (dashed) for a subject with no heart failure (No HF) and with heart failure (HFrEF), respectively, and
Fig.9 shows box plots of the indications of heart failure (HF-scores) for different sets of subjects in the evaluation of the proposed technology.

### Detailed description of drawings

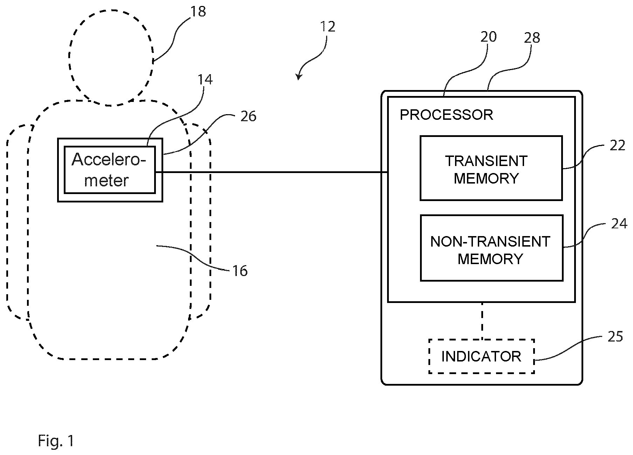

Fig.1 schematically illustrates an embodiment of a system 12 for determining an indication of heart failure of a subject 18. The system 12 has an accelerometer 14 in the form of a piezoelectric element that can be placed on the chest of a subject 18 and for measuring vibrations of the chest wall caused by movements of the heart. A processor 20 is connected with the accelerometer 14. The processor 20 has a transient memory 22 which can store a signal received from the accelerometer 14, and by which it can execute program code instructions. The system 12 comprises a support 26 that supports the accelerometer 14 and a housing 28 that accommodates the processor 20. The system 12 also has a non-transient memory 24 storing program code instructions for the processor 20. For example, the system 12 as a whole can be an integral part of a smartphone, or all parts except the accelerometer 20 and the support 26 can form part of a smartphone. In one embodiment, the accelerometer is an integrated accelerometer of a smartphone. In one embodiment of the system 12, it additionally has a display 25 that can display output information from the processor 20, such as a number.

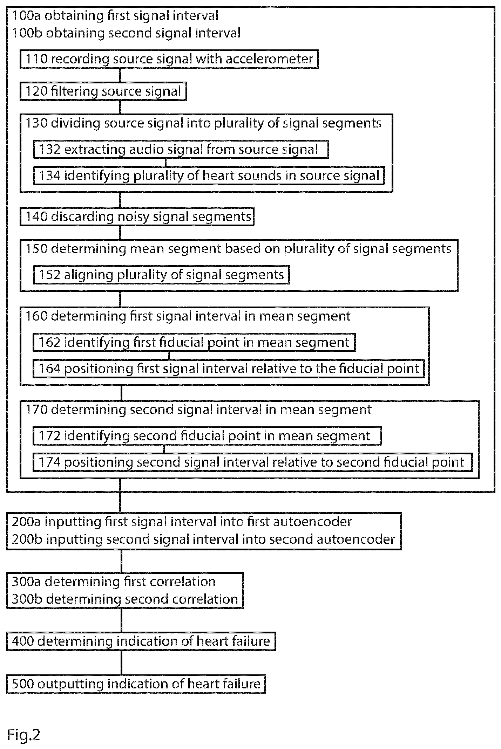

The program code instructions in the non-transient memory 24 cause the processor 20 to perform the method that is schematically illustrated in Fig.2. A source signal is recorded 110 with an accelerometer placed on the chest of a subject 18. The source signal is recorded over a period covering a plurality of cardiac cycles of the subject 18.

In an alternative embodiment (not shown), instead of the source signal being recorded, the source signal is downloaded from a computer server system (not shown) that is a general storage for source signals obtained by accelerometer placed on the chest of subject, for example for SCG and BCG purposes. This means that the source signal is not specifically intended for studying heart failure.

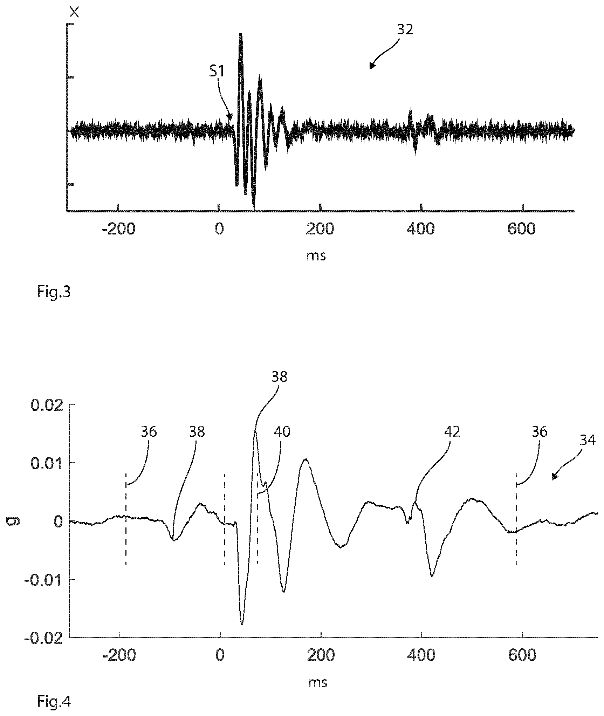

The source signal is filtered 120 with a high-pass filter having a cutoff frequency below **1.** The source signal is then divided 130 into a plurality of signal segments by extracting 152 an audio signal from the source signal using a high-pass filter having a lower cut-off frequency at 50 Hz. A portion of an audio signal 32 is shown in Fig.3. The abscissa represents the signal strength X (no unit) and the ordinate the time in milliseconds (ms). The S1 heart sounds are identified 134 in the audio signal, which are used to divide 130 the source signal into a plurality of signal segments such that each signal segment covers a single cardiac cycle. Noisy signal segments are discarded as specified in WO2017/216374 A1.

A mean segment 34 is then determined 150 based on plurality of signal segments by aligning 152 the plurality of signal segments using the S1 heart sounds associated with the signal segments. An example of a mean segment 34 is shown in Fig.4. The abscissa represents an acceleration in g (ms⁻²) and the ordinate the time in milliseconds (ms). Here, g is proportional to the voltage from the accelerometer 14.

A first signal interval 36 is obtained 100a by identifying 172 the Gs point in the systole as a first fiducial point 38 in the mean segment 34. The first fiducial point 38 is located in the systole. The first signal interval 36 extends from -250 to +500 ms relative to the first fiducial point 38. This means that the first signal interval 36 essentially covers the diastole of the mean segment 34 and corresponds to a first subinterval of the heart cycle. Similarly, a second signal interval 40 is obtained 100a by identifying 182 the Dd point in the diastole as a second fiducial point 42. The second signal interval 40 extends from -300 to +500 ms relative to the first fiducial point 38. Only the start of the second signal interval 40 is indicated in Fig. 4. This means that a second signal interval 40 essentially covers the systole of the mean segment 34.

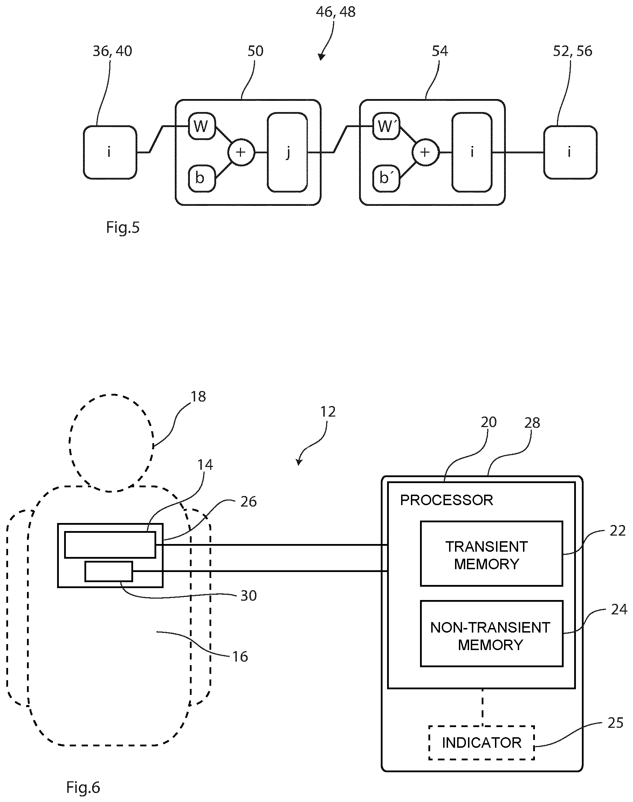

An autoencoder 46 and 48 is schematically illustrated in Fig.5. The autoencoder 46 and 48 has an encoder 50 that maps the input into the code, and a decoder 54 that maps the code to a reconstruction of the input. The input is a signal interval 36 and 40 having the sample length i. The encoder 50 maps the input down to j nodes. In the current embodiment, the number of nodes is eight and the sample length i is 401. The decoder 54 then maps the nodes to an output being a reconstructed signal intervals 52 and 56 having the same sample length i as the input. The weight matrices W and W' and the bias vectors b and b' have been determined by training the autoencoder 46 and 48 on healthy subjects.

The first signal interval 36 is inputted 200a into a first autoencoder 46 that has been trained on the corresponding first signal intervals from healthy subjects. The first autoencoder 46 outputs a reconstructed first signal interval 52. Similarly, the second signal interval 40 is inputted 200b into a second autoencoder 48. The second autoencoder 48 has been trained on the corresponding second signal intervals from healthy subjects. The second autoencoder 48 outputs a reconstructed second signal interval 56.

A first correlation between the first signal interval 36 and the reconstructed first signal interval 52 is determined 300a by calculating a first Pearson correlation coefficient for the two signals. Similarly, a second correlation between the second signal interval 40 and the reconstructed second signal interval 56 is determined 300b by calculating a second Pearson correlation coefficient for the two signals. In alternative embodiments, the MSE or RMSE between the signal intervals and the reconstructed signal intervals are calculated.

The indication of heart failure is determined 400 as a probability score for heart failure by a logistic regression model for the first and second Pearson correlation coefficients. Thus, the indication is based on the first correlation and the second correlation. The indication of heart failure is then displayed 500 on the display 25.

In an alternative embodiment of a system 12, no second signal interval is obtained and inputted in a second autoencoder. No second correlation is determined, and the indication of heart failure is based only on the first correlation.

Fig.6 schematically illustrates an alternative embodiment of a system for determining an indication of heart failure of a subject. The system 12 is similar to the system described in relation to Fig.1 and features with the same or related functions have the same number indices. Additionally, the system 12 has a microphone 30 in the form of a transducer that can convert sound into an electrical signal. The microphone 30 is supported by the support 26.

The program code instructions in the non-transient memory 24 correspond to those described in relation to Fig. 2 but differs in the dividing 130 of the source signal into the plurality of signal segments.

With the accelerometer 14 and the microphone 30 placed on the chest of the subject, the program code instructions cause the processor 20 to operate the microphone 30 to record an audio signal with the microphone 30 simultaneously to the source signal being recorded with the accelerometer 14. S1 heart sounds are identified in the audio signal. The source signal is divided into the plurality of signal segments based on the time correlation between the source signal, the audible signal, and the identified S1 heart sounds. The subsequent alignment of the plurality of signal segments is then based on the S1 heart sound in each signal segment.

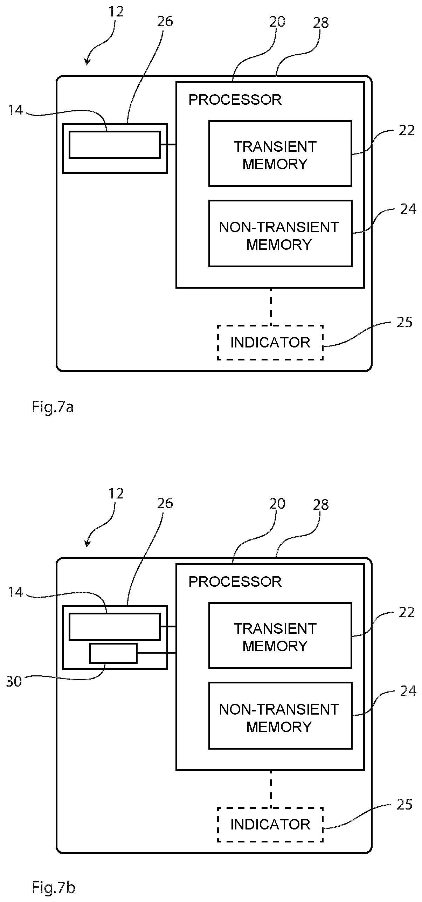

Fig.7a illustrates an alternative embodiment of the system 12 described in relation to Fig.1, with the only difference that the support 26 forms part of the housing 28 such that the housing 28 covers at least a portion of the accelerometer 14. In this embodiment, the housing 28 is placed on the chest of a subject, which means that the accelerometer 14 is also placed on the chest of a subject. Similarly, Fig.7b illustrates an alternative embodiment of the system 12 described in relation to Fig. 6, with the only difference that the support 26 forms part of the housing 28 such that the housing 28 covers at least a portion of the accelerometer 14 and the microphone 30. In this embodiment, the housing 28 is placed on the chest of a subject, which means that the accelerometer 14 and the microphone 30 are simultaneously placed on the chest of the subject.

### Proof of concept

A system was used based on the system described in relation to Figs.1 and 2. The method was developed to estimate the risk of having Heart Failure (HF) with low Ejection Fraction (HFrEF). The corresponding source signal is hereafter called an SCG signal.

SCG signals from 200 subjects were used for development and validation. The dataset was divided into a Training set and Test set with 100 subjects in each set.

**Table 1 shows baseline demographics and known HF diagnosis of the Training set and the Test set.**

| | **Training set** | **Test set** |
|---|---|---|
| **Total (N)** | 100 | 100 |
| **Gender (Male/Female)** | 45/55 | 51/49 |
| **Age in Years (Mean)** | 66.6 | 66.2 |
| **BMI(Mean)** | 30.7 | 29.5 |
| **Medical history:** | | |
| Diabetes (N) | 18 | 20 |
| Hypertension (N) | 62 | 63 |
| Coronary Artery disease(N) | 13 | 11 |
| Pulmonary disease(N) | 19 | 21 |
| Heart valve disease(N) | 2 | 5 |
| Oedemas (N) | 59 | 52 |
| **HF diagnosis** | | |
| No HF | 75 | 64 |
| Mild-HF | 21 | 19 |
| HFrEF | 4 | 17 |

The autoencoders were trained only on the 75 patients of the Training set with no heart failure diagnosis (No HF). Since the autoencoders were not trained to detect HFrEF, the autoencoders can be considered as unsupervised machine learning models.

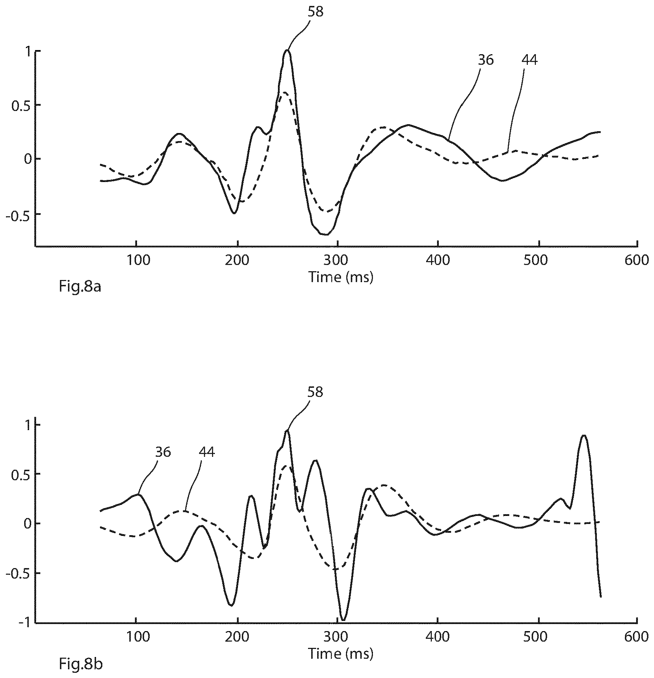

The first and second signal intervals of the 200 subjects were inputted in the trained first and second autoencoders, respectively, outputting first and second reconstructed signal intervals. An example of the first signal interval 34 solid and the reconstructed first signal interval 44 for a subject with No HF is shown in Figure Fig.8a. The corresponding signals for a subject with known HFrEF are shown in Fig.8b. The Gs points 58 are indicated in Figs. 8a and 8b. It can be seen in Figs.8a and b that the signal intervals and the reconstructed signal intervals differ more for the HFrEF subject than for the No HF subject. The first and second correlations were determined as described above in relation to Fig.2. Hereafter, the first correlation is denoted rSys and the second correlation is denoted rDia. It was found that the correlations rSys and rDia were higher for the No HF subjects than for the HFrEF subjects.

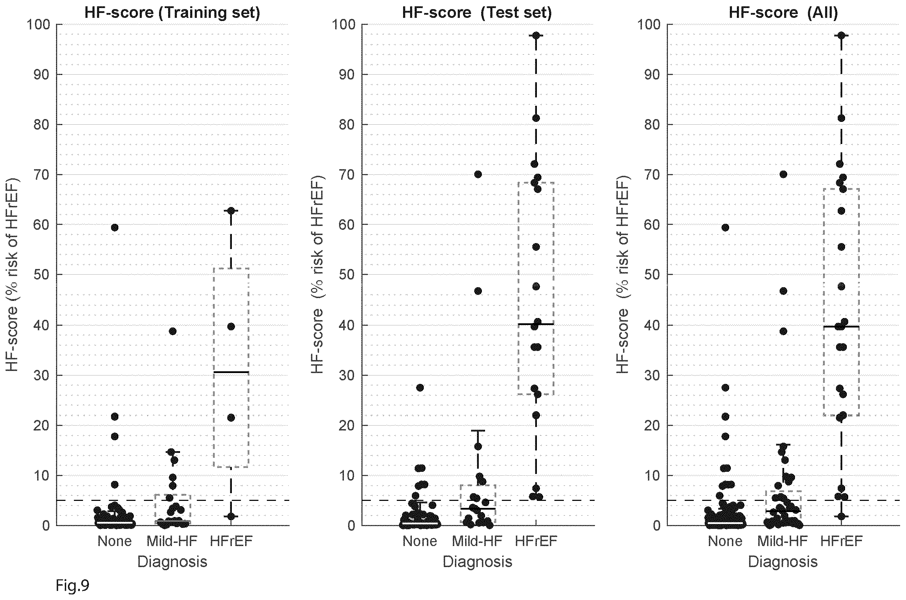

A logistic regression model was built for providing a risk prediction score based on the rSys and rDia. The risk prediction score is hereafter called HF-score and ranges from 0 to 100. The HF-score for the Training set, the Test set, and the combined sets (All) are shown in the box plots of Fig.9. A 5% risk threshold was defined as high risk for HFrEF, which is indicated by a horizontal dashed line.

**Table 3 showing the performance of the first autoencoder (rSys) and the second autoencoder (rDia) measured as the Area Under the receiver characteristic operator Curve (AUC).**

| | **All** | **Training set** | **Test set** |
|---|---|---|---|
| rSys AUC | 94.2% (87.2-100) | 91.9% (73.3-100) | 95% (87.6-100) |
| rDia AUC | 91.4% (83-99.8) | 85.2% (61.3-100) | 91.4% (82-100) |

**Table 4 showing the classification performance of the HF-score.**

| | **All** | **Training set** | **Test set** |
|---|---|---|---|
| N: Other | 179 | 96 | 83 |
| N: HFrEF | 21 | 4 | 17 |
| Prevalence of true (p=0.004012) | 10.5% | 4% | 17% |
| AUC (p=0.4135%) | 95.9% (89.9-100) | 92.7% (74.9-100) | 96.2% (89.8-100) |
| Negative predictive value (HF-score<=0.05)(p=0.3549) | 99.4% (96.5-100%) | 98.9% (93.8-100%) | 100% (94.8-100%) |
| Positive predictive value (HF-score>0.05) (p=0.05355) | 45.5% (30.4-61.2%) | 23.1% (5.04-53.8%) | 54.8% (36-72.7%) |
| Sensitivity (HF-score>0.05)(p=0.03465) | 95.2% (76.2-99.9%) | 75% (19.4-99.4%) | 100% (80.5-100%) |
| Specificity (HF-score<=0.05)(p=0.2691) | 86.6% (80.7-91.2%) | 89.6% (81.7-94.9%) | 83.1% (73.3-90.5%) |
| TN (HF-score<=0.05) | 155 | 86 | 69 |
| FN (HF-score<=0.05) | 1 | 1 | 0 |
| FP (HF-score>0.05) | 24 | 10 | 14 |
| TP (HF-score>0.05) | 20 | 3 | 17 |
| Likelihood ratio positive (HF-score> 0.05) | 7.103 | 7.2 | 5.929 |
| Likelihood ratio negative (HF-score<= 0.05) | 0.05499 | 0.2791 | 0 |

It was concluded that the AUC values obtain with both the autoencoders (rSys and rDia) and the classification performance of the HF-score confirm the strong HFrEF risk assent potential of the proposed technology.

## Claims

1. A system (12) for determining an indication of heart failure of a subject (18), wherein the system (12) comprises:
(A) an accelerometer (14) configured to be placed on the chest of a subject (18), and
(B) a processor (20) operatively connected to the accelerometer (14) and configured to perform a method for determining an indication of heart failure of the subject (18), wherein the method comprises:
- *obtaining* (100a) a first signal (36) interval from a source signal recorded with the accelerometer (14) placed on the chest of a subject (18), wherein the first signal interval (36) corresponds to a first subinterval of a heart cycle;
- *inputting* (200a) the first signal interval (36) into a first autoencoder, wherein the first autoencoder is trained on the corresponding first signal intervals obtained from healthy subjects and outputs a reconstructed first signal interval (44);
- *determining* (300a) a first correlation between the first signal interval (36) and the reconstructed first signal interval (44); and
- *determining* (400) the indication of heart failure based on the first correlation.

2. The system according to claim 1, wherein the first signal interval (36) covers a portion of the systole of the heart cycle.

3. The system according to claim 1 or 2, wherein the first autoencoder compresses the first signal interval (36) to a number of nodes, and the number of nodes is in the range 5 to 15, 6 to 12, or 7 to 9.

4. The system according to any of the claims 1 to 3, wherein the first autoencoder is a single layer autoencoder.

5. The system according to any of the claims 1 to 4, wherein the first correlation is a correlation measure between the first signal interval (36) and the reconstructed first signal interval (44), and the first correlation is based on a Pearson correlation coefficient, a Mean-Square Error (MSE), or a Root-Mean-Square Error (RMSE).

6. The system according to any of the claims 1 to 5, wherein the indication of heart failure is a probability score for heart failure, wherein the probability score is based on a logistic regression model and the first correlation.

7. The system according to any of the claims 1 to 6, wherein the method further comprises:
- *obtaining* (100b) a second signal interval (40) from the source signal recorded with an accelerometer (14) placed on the chest of a subject (18), wherein the second signal interval (40) corresponds to a second subinterval of a heart cycle;
- *inputting* (200b) the second signal interval (40) into a second autoencoder, wherein the second autoencoder is trained on the corresponding second signal intervals obtained from healthy subjects and outputs a reconstructed second signal interval (56); and
- *determining* (300b) a second correlation between the second signal interval (40) and the reconstructed second signal interval (56), wherein
determining (400) the indication of heart failure is further based on the second correlation.

8. The system according to any of the claims 1 to **7,** wherein obtaining (100a) the first signal interval (36) comprises:
- *recording* (110) a source signal with an accelerometer (14) placed on the chest of a subject (18), wherein the source signal is recorded over a period covering a plurality of cardiac cycles of the subject (18);
- *dividing* (130) the source signal into a plurality of signal segments, wherein each signal segment covers a single cardiac cycle;
- *aligning* (152) the plurality of signal segments;
- *determining* (150) a mean segment (34) based on, or from, the plurality of signal segments, and
- *determining* (160) the first signal interval (36) in the mean segment (34).

9. The system according to claim 8, wherein determining (160) the first signal interval (36) in the mean segment (34) comprises:
- *identifying* (162) a first fiducial point (38) in the mean segment (34); and
- *positioning* (164) the first signal interval (36) relative to the first fiducial point (38).

10. The system according to any of the claims 1 to 9, wherein the method further comprises: displaying the indication of heart failure.

11. The system (12) according to any of the claims 1-10, wherein the system (12) further comprises:
(C) a non-transient memory (24) storing program code instructions that, when executed by the processor (20), configures the processor (20) to perform the method.

12. A computer program product for being used in the system according to any of the claims 1-11, wherein the computer program product comprising program code instructions configured to, when executed by the processor (20) of the system (12), cause the processor (20) to perform the method.

13. A non-transient memory (24) on which a computer program product according to claim 12 is stored.

## Patentansprüche

1. System (12) zur Bestimmung eines Hinweises auf Herzinsuffizienz einer Testperson (18), wobei das System (12) Folgendes umfasst:
(A) einen Beschleunigungsmesser (14), der dafür konfiguriert ist, auf dem Brustkorb einer Testperson (18) platziert zu werden, und
(B) einen Prozessor (20), der operativ mit dem Beschleunigungsmesser (14) verbunden und dafür konfiguriert ist, ein Verfahren zur Bestimmung eines Hinweises auf Herzinsuffizienz der Testperson (18) durchzuführen, wobei das Verfahren Folgendes umfasst:
- *Erhalten* (100a) eines ersten Signalintervalls (36) aus einem Quellensignal, das mit dem auf dem Brustkorb einer Testperson (18) platzierten Beschleunigungsmesser (14) aufgezeichnet wurde, wobei das erste Signalintervall (36) einem ersten Teilintervall eines Herzzyklus entspricht;
- *Eingeben* (200a) des ersten Signalintervalls (36) in einen ersten Autocodierer, wobei der erste Autocodierer an den entsprechenden ersten Signalintervallen trainiert ist, die von gesunden Testpersonen erhalten wurden, und ein rekonstruiertes erstes Signalintervall (44) ausgibt;
- *Bestimmen* (300a) einer ersten Korrelation zwischen dem ersten Signalintervall (36) und dem rekonstruierten ersten Signalintervall (44); und
- *Bestimmen* (400) des Hinweises auf Herzinsuffizienz basierend auf der ersten Korrelation.

2. System nach Anspruch 1, wobei das erste Signalintervall (36) einen Teil der Systole des Herzzyklus abdeckt.

3. System nach Anspruch 1 oder 2, wobei der erste Autocodierer das erste Signalintervall (36) auf eine Anzahl von Knoten komprimiert und die Anzahl der Knoten im Bereich von 5 bis 15, 6 bis 12 oder 7 bis 9 liegt.

4. System nach einem der Ansprüche 1 bis 3, wobei der erste Autocodierer ein einschichtiger Autocodierer ist.

5. System nach einem der Ansprüche 1 bis 4, wobei die erste Korrelation ein Korrelationsmaß zwischen dem ersten Signalintervall (36) und dem rekonstruierten ersten Signalintervall (44) ist und die erste Korrelation auf einem Pearson-Korrelationskoeffizienten, einem mittleren quadratischen Fehler (MSE) oder einer Wurzel aus dem mittleren quadratischen Fehler (RMSE) basiert.

6. System nach einem der Ansprüche 1 bis 5, wobei der Hinweis auf Herzinsuffizienz ein Wahrscheinlichkeitswert für Herzinsuffizienz ist, wobei der Wahrscheinlichkeitswert auf einem logistischen Regressionsmodell und der ersten Korrelation basiert.

7. System nach einem der Ansprüche 1 bis 6, wobei das Verfahren ferner Folgendes umfasst:
- *Erhalten* (100b) eines zweiten Signalintervalls (40) aus dem Quellensignal, das mit einem auf dem Brustkorb einer Testperson (18) platzierten Beschleunigungsmesser (14) aufgezeichnet wurde, wobei das zweite Signalintervall (40) einem zweiten Teilintervall eines Herzzyklus entspricht;
- *Eingeben* (200b) des zweiten Signalintervalls (40) in einen zweiten Autocodierer, wobei der zweite Autocodierer an den entsprechenden zweiten Signalintervallen trainiert ist, die von gesunden Testpersonen erhalten wurden, und ein rekonstruiertes zweites Signalintervall (56) ausgibt; und
- *Bestimmen* (300b) einer zweiten Korrelation zwischen dem zweiten Signalintervall (40) und dem rekonstruierten zweiten Signalintervall (56), wobei
das Bestimmen (400) des Hinweises auf Herzinsuffizienz ferner auf der zweiten Korrelation basiert.

8. System nach einem der Ansprüche 1 bis 7, wobei das Erhalten (100a) des ersten Signalintervalls (36) Folgendes umfasst:
- *Aufzeichnen* (110) eines Quellensignals mit einem auf dem Brustkorb einer Testperson (18) platzierten Beschleunigungsmesser (14), wobei das Quellensignal über einen Zeitraum aufgezeichnet wird, der eine Vielzahl von Herzzyklen der Testperson (18) abdeckt;
- *Unterteilen* (130) des Quellensignals in eine Vielzahl von Signalsegmenten, wobei jedes Signalsegment einen einzelnen Herzzyklus abdeckt;
- *Ausrichten* (152) der Vielzahl von Signalsegmenten;
- *Bestimmen* (150) eines mittleren Segments (34) basierend auf oder aus der Vielzahl von Signalsegmenten, und
- *Bestimmen* (160) des ersten Signalintervalls (36) in dem mittleren Segment (34).

9. System nach Anspruch 8, wobei das Bestimmen (160) des ersten Signalintervalls (36) in dem mittleren Segment (34) Folgendes umfasst:
- *Identifizieren* (162) eines ersten Bezugspunkts (38) in dem mittleren Segment (34); und
- *Positionieren* (164) des ersten Signalintervalls (36) relativ zu dem ersten Bezugspunkt (38).

10. System nach einem der Ansprüche 1 bis 9, wobei das Verfahren ferner Folgendes umfasst: Anzeigen des Hinweises auf Herzinsuffizienz.

11. System (12) nach einem der Ansprüche 1-10, wobei das System (12) ferner Folgendes umfasst:
(C) einen nicht-flüchtigen Speicher (24), der Programmcodeanweisungen speichert, die bei Ausführung durch den Prozessor (20) den Prozessor (20) so konfigurieren, dass er das Verfahren durchführt.

12. Computerprogrammprodukt zur Verwendung in dem System nach einem der Ansprüche 1-11, wobei das Computerprogrammprodukt Programmcodeanweisungen umfasst, die so konfiguriert sind, dass sie bei Ausführung durch den Prozessor (20) des Systems (12) den Prozessor (20) veranlassen, das Verfahren durchzuführen.

13. Nicht-flüchtiger Speicher (24), auf dem ein Computerprogrammprodukt nach Anspruch 12 gespeichert ist.

## Revendications

1. Système (12) permettant de déterminer une indication d'insuffisance cardiaque d'un sujet (18), dans lequel le système (12) comprend :
(A) un accéléromètre (14) configuré pour être placé sur la poitrine d'un sujet (18), et
(B) un processeur (20) relié fonctionnellement à l'accéléromètre (14) et configuré pour réaliser un procédé de détermination d'une indication d'insuffisance cardiaque du sujet (18), dans lequel le procédé comprend :
- *l'obtention* (100a) d'un premier intervalle de signal (36) à partir d'un signal source enregistré avec l'accéléromètre (14) placé sur la poitrine d'un sujet (18), dans lequel le premier intervalle de signal (36) correspond à un premier sous-intervalle d'un cycle cardiaque ;
- *l'entrée* (200a) du premier intervalle de signal (36) dans un premier auto-codeur, dans lequel le premier auto-codeur est entraîné sur les premiers intervalles de signal correspondants obtenus à partir de sujets sains et produit un premier intervalle de signal reconstruit (44) ;
- *la détermination* (300a) d'une première corrélation entre le premier intervalle de signal (36) et le premier intervalle de signal reconstruit (44) ; et
- *la détermination* (400) de l'indication d'insuffisance cardiaque sur la base de la première corrélation.

2. Système selon la revendication 1, dans lequel le premier intervalle de signal (36) couvre une partie de la systole du cycle cardiaque.

3. Système selon la revendication 1 ou 2, dans lequel le premier auto-codeur compresse le premier intervalle de signal (36) en un certain nombre de nœuds, et le nombre de nœuds est dans la plage de 5 à 15, de 6 à 12 ou de 7 à 9.

4. Système selon l'une quelconque des revendications 1 à 3, dans lequel le premier auto-codeur est un auto-codeur à une seule couche.

5. Système selon l'une quelconque des revendications 1 à 4, dans lequel la première corrélation est une mesure de corrélation entre le premier intervalle de signal (36) et le premier intervalle de signal reconstruit (44), et la première corrélation est basée sur un coefficient de corrélation de Pearson, une erreur quadratique moyenne (MSE) ou une racine carrée de l'erreur quadratique moyenne (RMSE).

6. Système selon l'une quelconque des revendications 1 à 5, dans lequel l'indication d'insuffisance cardiaque est un score de probabilité d'insuffisance cardiaque, dans lequel le score de probabilité est basé sur un modèle de régression logistique et la première corrélation.

7. Système selon l'une quelconque des revendications 1 à 6, dans lequel le procédé comprend en outre :
- *l'obtention* (100b) d'un second intervalle de signal (40) à partir du signal source enregistré avec un accéléromètre (14) placé sur la poitrine d'un sujet (18), dans lequel le second intervalle de signal (40) correspond à un second sous-intervalle d'un cycle cardiaque ;
- *l'entrée* (200b) du second intervalle de signal (40) dans un second auto-codeur, dans lequel le second auto-codeur est entraîné sur les seconds intervalles de signal correspondants obtenus à partir de sujets sains et produit un second intervalle de signal reconstruit (56) ; et
- *la détermination* (300b) d'une seconde corrélation entre le second intervalle de signal (40) et le second intervalle de signal reconstruit (56), dans lequel
la détermination (400) de l'indication d'insuffisance cardiaque est en outre basée sur la seconde corrélation.

8. Système selon l'une quelconque des revendications 1 à 7, dans lequel l'obtention (100a) du premier intervalle de signal (36) comprend :
- *l'enregistrement* (110) d'un signal source avec un accéléromètre (14) placé sur la poitrine d'un sujet (18), dans lequel le signal source est enregistré sur une période couvrant une pluralité de cycles cardiaques du sujet (18) ;
- *la division* (130) du signal source en une pluralité de segments de signal, dans lequel chaque segment de signal couvre un seul cycle cardiaque ;
- *l'alignement* (152) de la pluralité de segments de signal ;
- *la détermination* (150) d'un segment moyen (34) basé sur, ou à partir de, la pluralité de segments de signal, et
- *la détermination* (160) du premier intervalle de signal (36) dans le segment moyen (34).

9. Système selon la revendication 8, dans lequel la détermination (160) du premier intervalle de signal (36) dans le segment moyen (34) comprend :
- *l'identification* (162) d'un premier point de repère (38) dans le segment moyen (34) ; et
- *le positionnement* (164) du premier intervalle de signal (36) par rapport au premier point de repère (38).

10. Système selon l'une quelconque des revendications 1 à 9, dans lequel le procédé comprend en outre : l'affichage de l'indication d'insuffisance cardiaque.

11. Système (12) selon l'une quelconque des revendications 1 à 10, dans lequel le système (12) comprend en outre :
(C) une mémoire non transitoire (24) stockant des instructions de code de programme qui, lorsqu'elles sont exécutées par le processeur (20), configurent le processeur (20) pour réaliser le procédé.

12. Produit de programme informatique pour une utilisation dans le système selon l'une quelconque des revendications 1 à 11, dans lequel le produit de programme informatique comprenant des instructions de code de programme configurées pour, lorsqu'elles sont exécutées par le processeur (20) du système (12), amène le processeur (20) à réaliser le procédé.

13. Mémoire non transitoire (24) sur laquelle est stocké un produit de programme informatique selon la revendication 12.
